(19) Europäisches Patentamt
European Patent Office
Office européen des brevets

(11) Numéro de publication: **0 023 178**
**B1**

## (12) FASCICULE DE BREVET EUROPEEN

(45) Date de publication du fascicule du brevet: **19.10.83**

(21) Numéro de dépôt: **80401069.2**

(22) Date de dépôt: **17.07.80**

(51) Int. Cl.³: **A 61 K 9/00,** A 61 K 31/70, C 07 H 15/18

(54) **Procédé de préparation de solutions concentrées d'amygdaline sous sa forme naturelle.**

(30) Priorité: **24.07.79 FR 7919076**

(43) Date de publication de la demande:
**28.01.81 Bulletin 81/4**

(45) Mention de la délivrance du brevet:
**19.10.83 Bulletin 83/42**

(84) Etats contractants désignés:
**AT CH DE GB LI NL**

(56) Documents cités:
**US - A - 3 734 902**

(73) Titulaire: **SOMET, Société dite**
**3, Rue de l'Industrie**
**(MC)**

(72) Inventeur: **Epuran, Jean**
**Sun-Tower**
**Monte-Carlo (MC)**

(74) Mandataire: **Ores, Irène et al,**
**CABINET ORES 6, Avenue de Messine**
**F-75008 - Paris (FR)**

**EP 0 023 178 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de solutions concentrées d'amygdaline sous sa forme naturelle

La présente invention est relative à un procédé de préparation de solutions concentrées de la forme naturelle de l'amygdaline.

L'amygdaline ou le mandélonitrile β-gentiobioside est un hétéroside (glucoside cyanogénétique) contenu dans l'amande amère et divers fruits d'amygdalacées et de pomacées.

Lorsque l'amygdaline est extraite du milieu naturel dans lequel elle se trouve et que l'on a pris toutes les précautions nécessaires pour qu'elle ne subisse aucune modification physico-chimique (et notamment aucune modification du pouvoir rotatoire) au cours de l'extraction et/ou de la purification, on obtient le D ou R mandélonitrile β-gentiobioside trihydraté qui répond à la formule générale I ci-après:

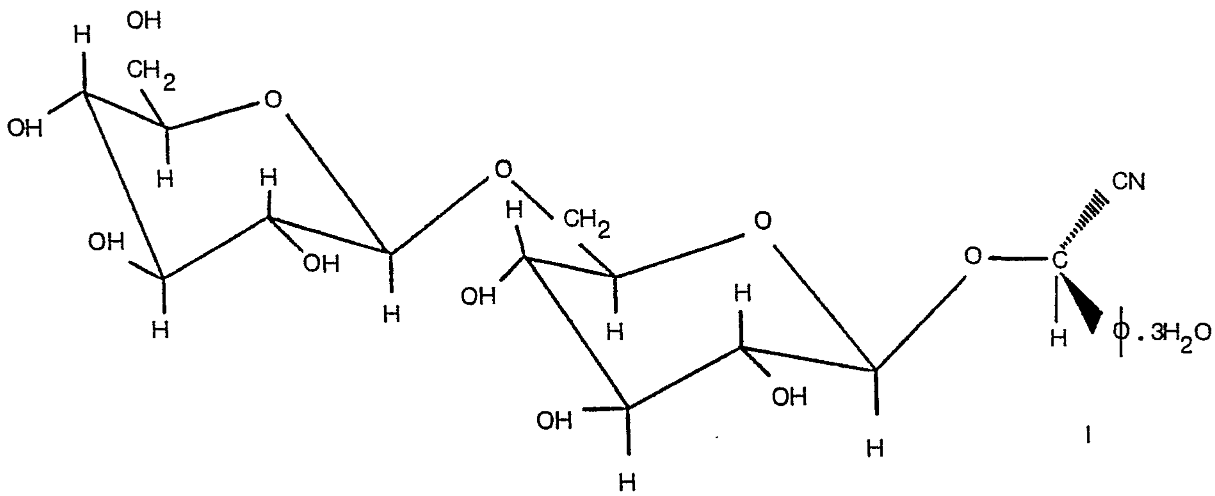

I

L'amygdaline constitue, comme on le sait, un cytostatique puissant, notamment à l'égard de cellules contenant l'enzyme β-glucuronidase qui hydrolyse l'amygdaline selon le schéma II ci-après:

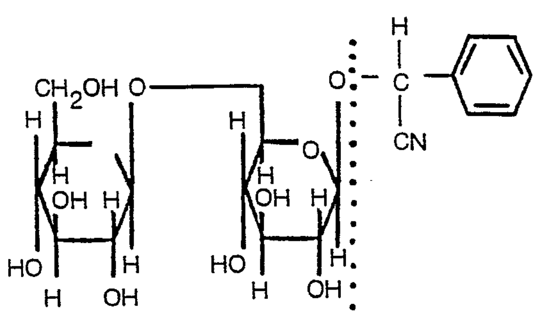

II

2 D GLUCOSE + 1 D MANDELONITRILE

Les quantités d'amygdaline qui doivent être administrées aux malades sont relativement importantes et s'élèvent à environ 20—25 g par jour. Comme la solubilité dans l'eau de l'amygdaline est assez faible, de l'ordre de 8 à 8,5% (1 g d'amygdaline se dissout dans environ 12 ml d'eau), ce sont donc des volumes importants qui sont injectés aux malades.

On a donc tenté, conformément à l'Art antérieur, d'augmenter la solubilité de l'amygdaline afin de pouvoir réduire les volumes injectés. Toutes ces tentatives, qui ont fait appel à des opérations physico-chimiques, ont donné lieu à des solutions d'amygdaline contenant une proportion élevée d'impuretés. Tel est le cas, par exemple, du procédé décrit dans le Brevet US—A—3 734 902, procédé qui consiste à dissoudre le produit dans un grand volume d'eau, puis à congeler rapidement la solution obtenue et à la lyophiliser.

Dans certaines de ces préparations, la teneur en amygdaline pure a même été réduite jusqu'à 65% par rapport à la quantité d'amygdaline pure initialement présente. C'est ainsi que 10 échantillons du commerce ont été analysés, et les résultats suivants ont été obtenus (Tableau I):

TABLEAU I

| LOT | $[\alpha]_D^{25}$ trouvé |
|---|---|
| 1 | −37°6 |
| 2 | −39°1 |
| 3 | −49° |
| 4 | −50°2 |
| 5 | −56°3 |
| 6 | −43°3 |
| 7 | −40° |
| 8 | −39°4 |
| 9 | −55° |
| 10 | −52° |

Or, la rotation optique de l'amygdaline pure est de −35°5 (C,1, eau) pour la forme trihydratée et de −42° (C,1, eau) pour la forme anhydre.

Lorsque l'amygdaline est dissoute dans l'eau, et qu'on soumet cette solution à l'action du temps, de la chaleur de la congélation, ou bien d'un alcali léger, ou bien encore de certains catalyseurs comme le noir animal, la forme optique initiale change brusquement, entraînant avec elle des modifications moléculaires, qui font suite à une ou plusieurs réactions chimiques d'hydrolyse donnant naissance à une ou plusieurs impuretés telles que l'amygdalinimide, le mandélonitrile ou encore le glucose.

Le mécanisme de l'action de ces divers processus physico-chimiques résulte des transformations qui s'opèrent sur le carbone assymétrique contenant les groupements nitrile, glucoside, hydrogène, benzène, lequel, au cours de la transformation optique, voit un de ses groupements s'échapper de la molécule, pour venir se placer dans une position symétrique. Au cours de cette réaction stéréochimique qui démarre à partir de l'épimère R pur (D mandélonitrile gentiobioside ou amygdaline de formule I), lequel se transforme pour une partie en épimère S (ou L mandélonitrile gentiobioside ou néo-amygdaline de pouvoir rotatoire $[\alpha]_D^{25} = -61°5$ C,1, eau), il se produit en plus, selon les conditions mises en oeuvre et selon la quantité d'épimère R transformée en épimère S, jusqu'à 30% de produits secondaires (amygdalinimides et même produits de l'hydrolyse du groupement nitrile ou de l'hydrolyse successive des groupements glucosido-glucosides). Cf. le schéma IV ci-après:

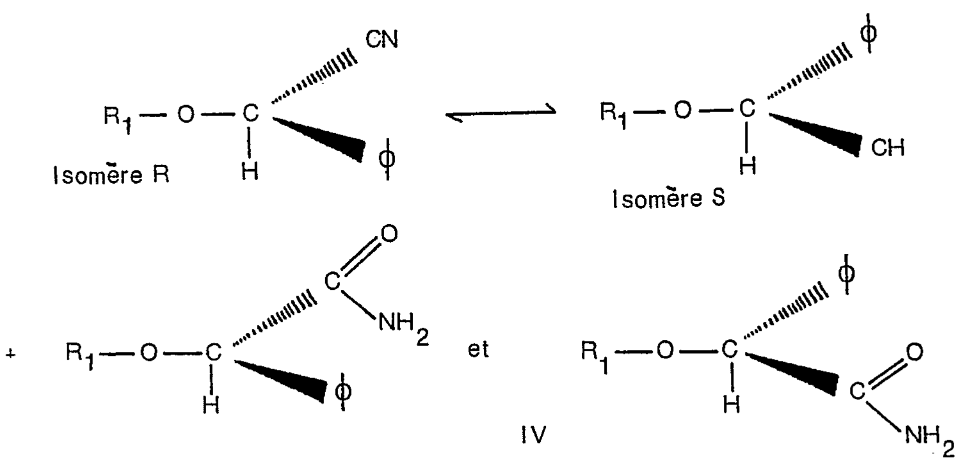

Isomère R

Isomère S

+

et

IV

$R_1$ étant le reste glucosido-glucosidique.

Alors que les solubilités des énantiomères sont identiques, celles des diastéréoisomères sont, d'une façon générale, différentes. C'est le cas pour le mélange épimère R et épimère S qui voit sa solubilité dans l'eau augmenter de quatre fois par rapport à l'épimère R seul.

Cette variation de solubilité a été mise à profit dans les préparations de l'Art antérieur, pour

dissoudre de grandes quantités de produit, mais si ces diastéréoisomères ont des propriétés chimiques ou physiques différentes, leurs propriétés pharmacologiques le sont aussi.

La présente invention s'est, en conséquence, donné pour but de pouvoir à des solutions concentrées d'amygdaline qui répondent mieux aux nécessités de la pratique que les solutions de l'Art antérieur, notamment en ce qu'elles rendent le traitement beaucoup plus supportable aux malades et ceci sans altérer en aucune manière les caractères physico-chimiques du produit naturel.

La présente invention s'est donné notamment pour but de préparer des solutions concentrées de la forme naturelle diastéréoisomère R qui répond à la formule D mandélonitrile $\beta$-D-glucosido-6-$\beta$-glucoside, de pureté optique maximum.

Au cours de travaux portant sur l'étude de la transformation de l'amygdaline en isoamygdaline, la Demanderesse a constaté qu'il y a migration de l'atome d'hydrogène du carbone assymétrique portant les groupements nitrile, benzyle et gentiobioside, avec formation d'un carbanion instable et fixation en position symétrique du proton, et ceci jusqu'à l'équilibre stable du mélange des deux diastéréoisomères R et S.

Autrement dit, afin d'empêcher cette migration et la formation d'isomère S, il y a lieu de favoriser la réaction inverse, c'est-à-dire de stabiliser la liaison C—H de la forme épimère R. La Demanderesse a pu mettre en évidence que cette stabilité est en relation directe avec le pH.

La présente invention a pour objet un procédé de préparation de solutions concentrées de la forme naturelle de l'amygdaline, caractérisé en ce que l'on dissout de l'amygdaline pure naturelle dans de l'eau, par chauffage rapide, à une température comprise entre 40°C et 115°C, ladite solution étant ajustée à l'aide des acides organiques pharmaceutiquement acceptables n'ayant pas d'atome d'halogène dans leur molécule, à un pH compris entre 3,5 et 4,5, et de préférence entre 3,7 et 3,9, après quoi on laisse refroidir la solution à la température ambiante.

Ainsi, en combinant l'effet de stabilisation du diastéréoisomère R aux pH acides, avec la propriété connue de l'amygdaline de présenter une solubilité importante aux températures élevées, on parvient à dissoudre une quantité importante d'amygdaline sans modifier la pureté optique initiale de l'amygdaline. En refroidissant ensuite, la température de la solution s'abaisse au-dessous de la valeur de saturation usuelle, sans que l'on observe un dépôt du produit. L'état de sursaturation ainsi obtenu peut être conservé autant de temps que l'on veut.

D'autre part, cette faculté de pouvoir chauffer à des températures élevées (jusqu'à 115°C) sans produire de diastéréoisomère S, permet en même temps de stériliser les solutions d'amygdaline sans altérer aucunement le produit.

Selon un autre mode de réalisation avantageux du procédé objet de la présente invention, la solution d'amygdaline est stérilisée avant refroidissement, en portant la température aux environs de 115°C pendant 15 à 25 minutes.

Conformément à l'invention, les solutions d'amygdaline ainsi obtenues peuvent avantageusement être mises sous forme de poudres par lyophilisation par congélation rapide et déshydratation sous vide.

La poudre ainsi obtenue est constituée par de l'amygdaline pure naturelle; elle se conserve pendant de longues périodes et conserve sa bonne solubilité.

L'invention pourra être mieux comprise à l'aide du complément de description qui va suivre, qui se réfère à des exemples de mise en oeuvre du procédé conforme à l'invention, à l'analyse des produits obtenus, ainsi qu'à une étude des caractéristiques de l'amygdaline en fonction du pH et de la température.

I. — ETUDE DE L'OBTENTION DE L'EQUILIBRE STABLE DES DIASTEREOISOMERES R $\rightleftharpoons$ S A 85°C EN FONCTION DU pH

Le Tableau II ci-après résume les résultats obtenus:

TABLEAU II

| pH solution | 3,8 | 4 | 4,5 | 5 | 6 | 7 |
|---|---|---|---|---|---|---|
| temps de chauffage à 85°C pour l'obtention de l'équilibre | 20 h | 15 h | 11 h | 2 h | 30 min. | 5 min. |

Il résulte très clairement de ces résultats que pour des chauffages rapides, le risque d'épimérisation est pratiquement nul pour des pH inférieurs ou égaux à 4.

II. — SOLUBILITE DE L'AMYGDALINE EN FONCTION DE LA TEMPERATURE

TABLEAU III

| Température | 5°C | 12°C | 22°C | 30°C | 40°C | 50°C | 80°C |
|---|---|---|---|---|---|---|---|
| Solubilité g/100 ml $H_2O$ | 4,5 | 5,5 | 8 | 14 | 25 | 36 | 60 |

La multiplicité des groupements hydroxyle confère à l'amygdaline une très grande solubilité à chaud.

En refroidissant ces solutions concentrées, on obtient des solutions sursaturées et parfaitement stables en fonction du temps, d'amygdaline pure naturelle. Ainsi, le traitement de malades se trouve considérablement amélioré (5 à 10 fois moins de volume à injecter).

Il est aisé de faire cesser cet état de sursaturation en projetant dans la solution un cristal d'amygdaline ou d'un corps isomorphe.

III. — EXEMPLES DE PREPARATION

Exemple 1

25 g d'amygdaline pure naturelle sont dissous dans 80 $cm^3$ d'eau bidistillée apyrogène, en chauffant à la température moyenne de 45°C. On ajuste le pH de la solution obtenue à 3,8 à l'aide d'acide glutamique et on filtre sur micropore 0,20 micron, puis on place la solution filtrée dans un ou plusieurs récipients préalablement stérilisés à 180°C pendant quatre heures; enfin les récipients sont fermés hermétiquement et stérilisés à 115°C pendant 20 minutes.

Après refroidissement à température ambiante, on obtient une solution limpide, claire, incolore, dépourvue d'impuretés mécaniques ou de cristallisation. Cette solution placée au réfrigérateur vers 0°C, ne cristallise pas. L'état de sursaturation ainsi produit permet la bonne conservation de l'amygdaline pure naturelle en solution aqueuse pendant au moins 20 mois.

Analyse: L'analyse est effectuée par comparaison simultanée du produit initial, cristallisé, et de l'amygdaline en solution.

Analyse de l'amygdaline cristallisée

I. — *Aspect:* Poudre blanche cristalline sans odeur.
Fusion köfler : vers 125°C (ramollissement)

II. — *Analyse spectrale:*
A. Spectre IR (KBr)
Pics maximum à 3300 $cm^{-1}$ ($OH_2$), 2250 $cm^{-1}$ (CN); 1650 $cm^{-1}$ ($OH_2$), 1450 $cm^{-1}$ 1380 $cm^{-1}$ (aryl) 1080—1020 (large) (C—OH) 760 et 705 $cm^{-1}$ (Ø).
B. Rotation optique (C,1,$H_2O$) $[\alpha]_D^{25} = -37°6$
C. Spectre UV (C.0,1,$H_2O$)
$\lambda$ max (nm) = 268, 262, 257, 252
titre obtenu par dosage UV = 105%

III. — *Chromatographie*
A. Chromatographie sur couche mince (Silicagel $F_{254}$Merck)
1. Solvant de migration:

| | |
|---|---|
| Acétate d'éthyle | 90 |
| Méthyléthylcétone | 54 |
| Acide formique | 18 |
| Eau distillée | 18 |

2. Quantité déposée
100$\gamma$ 200$\gamma$ ($H_2O$)
3. Détection — UV — Solution $\alpha$-naphtol
6% naphtol dans éthanol à 20%
Acide sulfurique au 1/2
4. Résultats
On peut observer la présence d'un seul spot à Rf: 0,20

B. Chromatographie en phase gazeuse

*Référence:* Par produit d'extraction — Somet CV 330

1. Colonne: L = 2m OV 17 3% sur Chromosorb W 100/120
2. Températures: Injecteur 300°C — Four 250°C. Détecteur 200°C
3. Gaz vecteur: Azote 1,45 kg/cm²
4. Détecteur: $H_2$-0,8 kg/cm² air 1,3 kg/cm²
5. Enregistreur: déroulement à 30 cm/heure
6. Quantité injectée: 0,1 $\mu$l dérivés sylilés
7. Résultats: le chromatogramme présente un seul pic à Tr = 29′ correspondant à l'amygdaline épimère R pur.

*Résonnance magnétique nucléaire*

Référence: TMS    Solvant $D_2O$

Le spectre obtenu correspond à la structure avec déplacement chimique ($\delta$)

| | | groupement |
|---|---|---|
| 3,4 — 4,4 | | protons glucosides |
| 5,8 | singulet | H—C—C≡N (forme D) |
| 7,35 | singulet | protons groupe phényle |

L'amygdaline considérée correspond à l'épimère R pur.

Analyse de l'amygdaline mise en solution selon l'Exemple 1

I. — *Aspect:* Solution limpide, incolore, sans impuretés mécaniques ni cristallisations.

Par évaporation au bain-marie à 30°C sous 2 mm Hg, résidu cristallisé blanc — Point de fusion: vers 125°C

II. — *Analyse spectrale* (effectuée sur le résidu cristallisé blanc):

A. Spectre IR (KBr)

Pics maximum à 3300 $cm^{-1}$, 2250, 1650, 1450, 1380, 1080, 1020 (large) 760 et 705 $cm^{-1}$.

B. Rotation optique: (C,1,$H_2O$) $[\alpha]_D^{25} = -40°6$ (Dilution c=1 $H_2O$ obtenue sur la base de la concentration de la solution préparée) C=25%

C. Spectre UV (C, 0,1 $H_2O$)

λmax (nm): 268, 262, 257, 262

Titre obtenu par dosage UV: 110%

(Dilution pour C=0,1 sur la base de la concentration c = 25% de la solution préparée).

III — *Chromatographie*

A. Chromatographie sur couche mince: mêmes caractéristiques techniques.

1. Quantité déposée. 100$\gamma$ 200$\gamma$
2. Référence: le produit cristallise avant dissolution
3. Résultats: l'amygdaline en solution présente le même spot que l'amygdaline initiale Rf = 0,20

B. Chromatographie en phase vapeur: mêmes caractéristiques techniques.

Dérivés triméthylsililés sur produit d'évaporation

Référence: chromatogramme amygdaline avant conditionnement.

*Résultats:* un seul pic amygdaline Tr = 29 minutes correspondant à l'épimère R pur.

Exemple 2

De la même façon que dans l'Exemple 1, 16,5 grammes d'amygdaline pure naturelle, sont dissous dans 88 cm³ d'eau bidistillée apyrogène.

Le pH de cette solution est ajusté à 3,8 par addition de 10 mg d'acide glutamique pur cristallisé. La solution est filtrée sur Micropore 0,20 micron, puis répartie dans divers récipients préalablement stérilisés à 180°C pendant 4 heures.

La solution conditionnée dans les récipients correctement fermés est autoclavée à 115°C pendant 20 minutes; on obtient après refroidissement, une solution limpide et claire, dépourvue d'impuretés mécaniques ou de cristallisation. La solution étant stérile, elle peut se conserver pendant au moins 20 mois.

Analyse: Résultats

L'analyse effectuée comparativement avec le produit initial cristallisé montre que l'amygdaline en solution a gardé son identité structurale et sa qualité optique. Cette analyse est effectuée successivement par l'analyse spectrale (IR UV $[\alpha]_D$) et l'analyse chromatographique (CCM, CPV).

Exemple 3

10 grammes d'amygdaline pure naturelle sont dissous à la température de 30°C dans 90 grammes d'eau bidistillée apyrogène.

Le pH de la solution obtenue est ramené au voisinage de 3,8 par addition de 15 mg d'acide glutamique, puis filtrée sur Micropore 0,20 $\mu$ et répartie par fractions de 10 ml dans des flacons de 20 ml.

Chaque flacon est bouché partiellement par un tampon biologique, puis est soumis à un autoclavage de 115°C pendant 10 minutes. Après refroidissement, la solution est soumise à une rapide congélation vers −45°C; −50°C, puis déshydratée par le vide. Le lyophilisat obtenu correspond à de l'amygdaline pure naturelle.

Analyse

I. *Aspect:* Poudre blanche hygroscopique
Fusion köfler: 120°C avec ramollissement

II. *Analyse spectrale:*
A. — Spectre IR: conforme à la structure
B. — Rotation optique: $[\alpha]_D^{25} = -38°$
C. — Spectre UV: $\lambda$ (max) = 268, 262, 257, nm
252, titre obtenu par dosage UV = 102%

III. *Chromatographie:*
A. — sur couche mince (Silicagel)
1 seul spot identique au produit initial
Rf = 0,20
B. — en phase vapeur
1 seul pic à Tr = 29 minutes conforme à l'épimère R pur

Il résulte de la description qui précède que, quels que soient les modes de mise en oeuvre, de réalisation et d'application adoptés, l'on obtient des solutions concentrées, de toutes concentrations désirées et contrôlées, d'amygdaline naturelle pure, dépourvue d'impuretés et stérile, lesquelles solutions concentrées peuvent, si on le désire, être stockées (telles quelles ou à l'état lyophilisé) pendant de longs mois.

**Revendications**

1. Procédé de préparation de solutions concentrées de la forme naturelle de l'amygdaline, caractérisé en ce que l'on dissout de l'amygdaline pure naturelle dans de l'eau, par chauffage rapide, à une température comprise entre 40°C et 115°C, ladite solution étant ajustée à l'aide des acides organiques pharmaceutiquement acceptables n'ayant pas d'atome d'halogène dans leur molécule, à un pH compris entre 3,5 et 4,5, après quoi on laisse refroidir la solution à la température ambiante.

2. Procédé selon la Revendication 1, caractérisé en ce que le pH est ajusté à une valeur comprise entre 3,7 et 3,9.

3. Procédé selon l'une quelconque des Revendications 1 et 2, caractérisé en ce qu'on stérilise la solution d'amygdaline avant refroidissement, en portant la température de ladite solution aux environs de 115°C, pendant 15 à 25 minutes.

4. Procédé selon la Revendication 1, caractérisé en ce que l'acide utilisé est de l'acide glutamique.

**Claims**

1. Process for the preparation of concentrated solutions of the natural form of amygdalin, wherein pure natural amygdalin is dissolved in water, by rapid heating, at a temperature comprised between 40°C and 115°C, said solution being adjusted by means of pharmaceutically acceptable organic acids not having a halogen atom in their molecule, at a pH comprised between 3.5 and 4.5, after which the solution is left to cool to ambient temperature.

2. Process according to claim 1, wherein the pH is adjusted to a value comprised between 3.7 and 3.9.

3. Process according to any one of claims 1 and 2, wherein the amygdalin solution is sterilised before cooling, by bringing the temperature of said solution to about 115°C, for 15 to 25 minutes.

4. Process according to claim 1, wherein the acid used is glutamic acid.

**Patentansprüche**

1. Verfahren zur Herstellung von konzentrierten Lösungen der natürlichen Form von Amygdalin, dadurch gekennzeichnet, daß man reines natürliches Amygdalin in Wasser durch rasches Erwärmen bei einer Temperatur von 40°C bis 115°C auflöst, die Lösung mit Hilfe von pharmazeutisch brauchbaren organischen Säuren, die kein Halogenatom in ihrem Molekül aufweisen, auf einen pH-Wert von 3,5 bis 4,5 einstellt und anschließend die Lösung auf Umgebungstemperatur abkühlen läßt.

2. Verfahren nach Anspruch 1, dadurch gekennzeichnet, daß der pH-Wert auf einen Wert von 3,7 bis 3,9 eingestellt wird.

3. Verfahren nach einem der Ansprüche 1 und 2, dadurch gekennzeichnet, daß man die Amygdalin-Lösung vor dem Abkühlen sterilisiert, wobei man die Temperatur dieser Lösung während 15 bis 25 min auf etwa 115°C bringt.

4. Verfahren nach Anspruch 1, dadurch gekennzeichnet, daß die verwendete Säure Glutaminsäure ist.